# EUROPEAN PATENT APPLICATION

(11) **EP 4 714 463 A1**
(43) Date of publication of application: **25.03.2026**
(21) Application number: 24739076.8
(22) Date of filing: 10.05.2024
(51) Int. Cl.: A61K 39/12, A61P 31/14, A61P 31/20

(54) **FORMULATION FOR IMMUNIZATION OF PIGS BY THE ORAL ROUTE**

(30) Priority: 18.05.2023 CU 20230025
(71) Applicant: Centro de Ingeniería Genética y Biotecnología, La Habana 11600 (CU)
(72) Inventor: SORDO PUGA, Yusmel, La Habana 10900 (CU); RODRÍGUEZ MOLTÓ, María Pilar, La Habana 11600 (CU); PÉREZ PÉREZ, Danny, La Habana 11600 (CU); SUÁREZ PEDROSO, Marisela Fátima, La Habana 11600 (CU); ESTRADA GARCÍA, Mario Pablo, La Habana 11600 (CU); DUARTE CANO, Carlos Antonio, La Habana 11300 (CU); SARDINA GONZÁLEZ, Talía Caridad, La Habana 10400 (CU); HERNÁNDEZ GARCÍA, Mara Laura, La Habana 11300 (CU); MÉNDEZ ORTA, Mary Karla, La Habana 11300 (CU); RODRÍGUEZ MALLÓN, Alina, La Habana 11300 (CU); LAO GONZÁLEZ, Thailin, La Habana 11300 (CU)
(74) Representative: V.O.
(86) International application number: PCT/CU2024/050003
(87) International publication number: WO 2024/235366

(57) **Abstract**

An immunogenic formulation by the oral route comprising: i) one or several chimeric proteins consisting of an immunologically relevant antigen from a pig's pathogen microorganism and the molecular adjuvant CD154 from swine, ii) an adjuvant selected from the cyclic di-nucleotide monophosphate family, and iii) an excipient acceptable for oral immunization. A method to induce an immune response against pathogens, characterized by the administration by the oral route of an immunogenic formulation comprising one or several chimeric proteins consisting of i) an immunologically relevant antigen from a pig's pathogen microorganism fused to the molecular adjuvant CD154 from swine, ii) and an adjuvant selected from the family of the cyclic di-nucleotide monophosphate.

## Description

### Field of the present invention

The present invention relates to the fields of virology a, vaccinology, and biopharmaceutical industry. In particular, it discloses vaccine formulations combining one or several chimeric proteins, consisting of an immunologically relevant antigen from a pig's pathogen microorganism fused to the molecular adjuvant CD154 from swine, with the adjuvant cyclic di-adenosine monophosphate (c-di-AMP) for the oral immunization in pigs.

### State of the art

The development of oral vaccines shows evident practical advantages in comparison to traditional parenteral vaccines. Among these advantages are the lower cost, greater safety, ease in the application and greater adherence of people, as well as the possibility of inducing both, systemic and mucosal immune responses (Ou B et al., BioDrugs. 2023 Mar;37(2):143-180.).

However, the development of oral vaccines is quite a challenge for two main motives: 1) It is necessary to protect immunogenic antigens from the very harsh environment of the digestive tract, and 2) It is difficult to bypass the tolerogenic mechanisms associated to the ingestion of proteins. For these reasons, the only oral vaccines developed so far has been live attenuated vaccines (LAV) such as the Sabin vaccine against poliomyelitis in humans.

In the field of veterinary vaccines, particularly in vaccines aimed to prevent pig's diseases, oral LAV against Classical Swine Fever (CSF), a highly contagious viral disease with a severe socioeconomic impact in the world pig's industry, have also been explored with some degree of success (Ma et al., Genetics and Molecular Res. 2014; 13: 10803-10810). The etiological agent of CSF is the Classical Swine Fever Virus (CSFV), a member of the Pestivirus genus, *Flaviviridae* family (Simmonds et al., J. Gen. Virol. 2017; 98, 2: 2).

In this case, an oral vaccine is required for the immunization of wild pigs and boars, which are important reservoirs of the virus, even in countries where no circulation of the virus exist among domestic pigs. Those wild animals cannot be vaccinated like farm pigs; therefore, another approach is required to protect them from the disease and avoid the dissemination of the virus towards production farms. With these purposes, LAV vaccines delivered in bites have been scattered through the habitat of wild boars for the oral ingestion by these animals (Milicevic et al., Veterinary Microbiol., 2013; 163: 167-171; Von Rüden et al., Veterinary Microbiology 2008; 132(1-2): 29-38). Nevertheless, the regulatory authorities are increasingly reluctant to release attenuated viruses in the environment that could eventually reverse toward a pathogenic phenotype and lead to new outbreaks of the disease. Furthermore, the selective pressure resultant from the prolonged use of LAV in endemic regions selects for viral strains of low or intermediate virulence (Coronado et al., Transboundary and Emerging Diseases 2019; 66: 2362-2382), which originates from recombination or mutation events conferring the capacity to escape from the host immune response (Hu et al., Frontiers in microbiology, 2016, 7: 85; Ji et al., Journal of Molecular Epidemiology and Evolutionary Genetics in Infectious Diseases 2014; 25: 69-77). As a result of this adaptive process, mild and moderated infections are established, where clinical sigs are often masked. The accurate diagnosis of these cases is not feasible, since diagnostic tests cannot discriminate between antibodies elicited by the vaccine and those induced in infected individuals.

Other limitation of oral vaccines based on LAV is the dependence from a cold chain, since these vaccines are sensitive to temperature changes and need to be stored and transported between 4°C and 8°C. Finally, but not less relevant, the colostrum neutralizing antibodies (CNAb), passively transferred from the vaccinated mother to the piglets interfere with the replication of LAV, and, therefore, cause a drastic reduction in their immunogenicity (Blome et al., Viruses 2017; 9: 86).

To overcome these difficulties, efficient subunit vaccines, which could be used in elimination campaigns in endemic regions, or in emergencies to control outbreaks in CSFV free regions, must be developed (Blome et al., Veterinary Microbiology 2017; 206:10-20).

The knowledge about the bases of the protective immunity against CSFV has led to the development of subunit vaccine candidates as an attractive alternative to LAV for the control and elimination of CSF (Darwich et al., J Gen Virol. 2003; 84: 3453-3457; Dong et al., Vaccine 2007; 25: 205-230). The E2 glycoprotein of the viral envelope has been the more frequent election to induce a protective response against CSFV (Madera et al., BMC Veterinary Research 2016; 12: 197; Toledo et al., J Biotechnol. 2008; 12: 370-376). These vaccines are safe and induce a potent neutralizing antibody (NAb) response (Barrera et al., Vet Immunol Immunopathol. 2009; 127: 325-331). Additionally, subunit vaccines allow to discriminate infected from vaccinated animals (DIVA) (Dong et al., Vaccine 2007; 25: 205-230). However, marker vaccines based of E2 evaluated so far, have only conferred a late onset of protection against infection and disease, which is a disadvantage in comparison with LAV (Hua et al., PLoS One 2014; 9: e106891; Bouma et al., Vaccine 2000; 18: 1374-1381). These vaccines have not been neither successful in preventing the vertical transmission of CSFV from infected mothers to their offspring, which is another important limitation because piglets perennially infected are born.

Up to date there is not subunit vaccine against CSFV administered by the oral route in pigs.

CD154 molecule (CD40 ligand) is a glycoprotein form the tumor necrosis factor family. The binding of CD154 in the surface of T cells to its CD40 ligand is essential to promote and potentiate the humoral and cellular immune response (Hashem et al., J. Immunol. 2014; 193: 722-734). The interaction of CD154 with antigen presenting cells stimulates the expression of co-stimulatory and adhesion molecules, and the positive regulation of cytokine secretion (Trella et al., Eur. J. Immunol. 2016; 46: 420-431). Because of these properties, CD154 molecule has been used as a molecular adjuvant to potentiate the immunogenicity of subunit vaccines against different viral diseases (Cao et al., Vaccine 2010; 28: 7514-7522; Tang, et al., Cancer Immunol Immunother 2009; 58: 1949-1957).

The chimeric protein E2CD154 was developed by fusing the E2 glycoprotein of CSFV and the pig CD154 antigen to overcome the drawbacks of both types of existent vaccines (Toledo et al., WO/2007/098717 A2). This protein, when administered by the systemic route in an emulsion with the oil adjuvant Montanide ISA50 V2, confers an early onset of protection against the viral challenge with a highly virulent strain of CSFV (Suárez et al., Vaccine 2021; 35(34): 4437-4443; Sordo-Puga et al., Vaccines, 2021; 9, 2: 167). Moreover, it is capable of preventing the vertical transmission of the virus (Muñoz-González et al., Vet. Microbiol. 2017; 205: 110-116). However, this antigen did not generate a detectable immune response in pigs, either systemic or mucosal, when administered orally in repeated high doses.

Another viral disease that is a growing threat to the global swine industry is African Swine Fever (Rock DL. Viruses 2021; 13(5): 943). Its etiological agent is the African Swine Fever virus (ASFV), the only known member of the *Asfarviridae* family. Although to date no vaccine with proven efficacy against this disease has been developed, evidence of partial protection has been collected by immunization with LAV (Chen et al., Sci China Life Sci. 2020;63(5):623-634) or combinations of different ASFV antigens. Among the most promising proteins as vaccine antigens are the p72 and p30 capsid proteins, capable of inducing a neutralizing antibody response in pigs. (Murgia et al., Archives of Virology, 2019; 164:359-370, Kollnberger et al., J Gen Virol. 2002; 83:1331-42).

The ideal oral vaccine against these and other swine pathogens would be a non-replicating subunit vaccine that does not carry any risk such as those described above for CSF. To achieve this, it is necessary to overcome the challenge that non-replicating antigens administered by the oral route generally induce tolerance and not immunity (Strobel et al., Immunology Today, 1998; 19: 173-181; Zhu y Berzofsky, Gut Microbes 2013; 4: 246-252).

For these reasons, there are currently no registered subunit vaccines for oral use in either humans or animals. The only approved oral vaccines are based on attenuated rotavirus, polio viruses, or fully inactivated *Vibrio cholerae* bacteria (Marasini et al., Expert Review of Vaccines 2014; 13: 1361-1376).

Numerous studies have been conducted in animal models with the aim of developing adjuvants capable of promoting a more efficient response against protein antigens administered by the mucosal route. The effects sought with these adjuvants, vehicles, or immunomodulators include: 1) protecting vaccine antigens from the action of proteases during their passage through the digestive tract, 2) facilitating the adhesion of these antigens to the mucosal membranes, and 3) stimulating the receptors of the immune system to induce an effective immune response. Among the compounds evaluated as adjuvants are bacterial toxins and their derivatives (Pizza et al., Vaccine. 2001;19(17-19):2534-2541; Inskeep TK, et al., Clin Vaccine Immunol. 2010; 17(8): 1163-1169; McCluskie et al., Vaccine, 2000; 19: 950-957), and oligonucleotides containing CpG motifs (McCluskie et al., Vaccine 2000; 19: 950-957), among others. The encapsulation of antigens in nanoparticles composed of compounds of various chemical nature has also been tested (Minato et al., Journal of Controlled Release 2003; 89: 189-197; Rezaei et al., Biomaterials Science 2021; 9: 2679-2695; Tian et al., International Immunopharmacology 2008; 8: 900-908; Salman et al., Vaccine, 2009; 27: 4784-4790; Slütter et al., Journal of Controlled Release 2009; 138: 113-121; Jain et al., International Journal of Pharmaceutics 2010; 387: 253-262; Sarti et al., Biomaterials 2011; 32: 4052-4057). However, despite promising results in murine models, these formulations have not been able to become useful products for humans or animals.

One of the adjuvants that has been successfully used to enhance the immune response against different antigens, both systemically and mucosally, are the so-called cyclic di-nucleotides (CDN) (Ebensen et al., Clin. Vac. Immunol. 2007; 14 (2007) 952-958; Ebensen et al., Frontiers in Immunology 2017; 8:1223), and in particular the cyclic di-adenosine monophosphate (c-di-AMP) (Ebensen et al., Vaccine 2011; 29 (32): 5210-5220). These molecules of bacterial origin function as agonists of Sting receptors (Stimulator of Interferon Genes), which mediate the activation of different cellular processes related to the activation of type 1 interferon secretion in immune system cells (Yan, H. & Chen, W. Vaccines 2021; 9: 917). However, most of these experiments have been conducted in mice, a model very distant from larger animal species such as pigs or humans. For this reason, it is not possible to automatically extrapolate the results from one species to another.

The combination of cyclic di-guanosine monophosphate (c-di-GMP) with inactivated influenza virus led to an increase in antibody response when administered intradermally (ID) in pigs (Wang et al., 2016, Journal of Investigative Dermatology, 136: 2183-2191). The addition of c-di-GMP along with trehalose 6,6'-dibenzene (TDB), an agonist of Mincle receptors, significantly increased the antibody response against a mixture of foot-and-mouth disease virus antigens also adjuvanted with AlOH and Quil A in pigs, but, in this case, the immunogen was administered by the intramuscular route (Lee et al., Frontiers in Immunology 2019;10: 2509).

The addition of c-di-AMP increased both the antibody response and that of T cells in combination with *Mycoplasma hyopneumoniae* bacterins, administered by the intradermal route (ID) in pigs (Matthijs et al., 2019, Frontiers in Immunology, 10: 1087). Also, by the ID route, the combination of c-di-AMP with nanoparticles between 70 and 80 nm increased both humoral and cellular response in pigs against ovalbumin.

However, none of these investigations verified the functionality of the immune response induced by these immunogens, which is a major limitation. On the other hand, the addition of c-di-AMP to an influenza peptide administered by ID route in pigs induced low levels of antibodies but was not able to protect animals from a challenge with a pathogenic strain. This indicates that the antibodies generated were not functional; on the contrary, post-challenge viral load was higher in vaccinated animals than in controls, so the effect obtained was opposite to that desired (López-Serrano et al., Vaccines 2021;9: 751).

The only attempt to use CDN as an adjuvant by the nasal route in pigs, by mixing it with virus-like particles from porcine reproductive and respiratory syndrome virus (PRRSV), not only failed to break the tolerance as no antibodies against the virus were detected, but induced a status of greater susceptibility of animals to a challenge with a pathogenic strain (Van Noort et al., Virology Journal https://www.deepl.com/es/translator 2017; 14:1-9).

The enumerated results indicate that oral immunization of pigs with subunit vaccines is an unresolved problem, and that experiments conducted in mice, or using other routes of immunization, are not automatically extrapolable to immunization by the oral route in this species.

### Explanation of the invention

The present invention is aimed at solving the problem posed by the absence of effective oral subunit vaccines for pigs. This application shows examples of oral formulations against classical swine fever and African swine fever, although it is not limited to these diseases, as the vaccine formulations disclosed can also be used against other swine pathogens.

In its most general approach these vaccine formulations comprise one or several chimeric proteins formed by i) an immunologically relevant antigen of a pig pathogen microorganism fused to the molecular adjuvant CD154 from swine, and ii) an adjuvant selected from the family of cyclic di-nucleotide monophosphates, for oral immunization of pigs. A preferred embodiment of the invention is the combination of the E2CD154 antigen, formed by the fusion between the E2 protein of CSFV and the porcine CD154 molecule (SEQ ID NO: 1), with the adjuvant cyclic di-adenosine monophosphate (c-di-AMP), and this combination is administered to pigs orally, either in solution, encapsulated in nanoparticles, or together with a pharmaceutically acceptable excipient.

In particular, the invention relates to the administration of this mixture of E2CD154 and c-di-AMP to pigs through a bait, which can be based on different types of food, such as rice, wheat flour, corn, or other cereals, so that it is attractive for consumption by wild animals.

The present invention demonstrates that E2CD154 protein identified in Sequence Listing as SEQ ID NO:1 is surprisingly capable of inducing a potent immune response when administered orally in mice in combination with c-di-AMP adjuvant.

In the first example of the present invention, the immunogenicity of the combination of the protein identified as SEQ ID NO: 1, and the adjuvant c-di-AMP administered orally in mice is demonstrated. This immune response was characterized by the appearance of specific antibodies in serum. It was also demonstrated that these antibodies are functional, as they are capable of neutralizing the replication of CSFV with titers above 1:50 in a peroxidase-linked neutralization assay (NPLA).

In example 2 of the present invention, it is also demonstrated that this combination of c-di-AMP and the molecular adjuvant CD154 is key to achieving a potent humoral response against the vaccine antigen. Both immunomodulatory elements act synergistically, as the combination of E2 protein without CD154 with c-di-AMP does not induce the same antibody response in mice, neither in frequency nor in the antibody titers achieved.

In example 3, it is illustrated how a formulation comprising the protein identified in the Sequence List as SEQ ID NO: 1 and c-di-AMP, administered orally to pigs, induced specific antibodies against E2 in serum, and these antibodies in turn were capable of neutralizing CSFV replication in an NPLA assay with titers of 1:12.5 and 1:25.

In example number 4 of the present invention, it is also evidenced that, surprisingly, oral administration of the formulation described in the present invention was able to protect these pigs from a lethal challenge with the highly virulent strain "Margarita", belonging to subgenotype 1.4 of CSFV. This result is unexpected and contrasts with previous experiments described in the literature where other authors had not only found no protective effect after immunization with formulations containing this type of adjuvant but, on the contrary, its administration had increased susceptibility of animals to infection (Van Noort et al., 2017, Virology Journal, 14:1-9). Three out of four animals vaccinated with higher doses of E2CD154 and c-di-AMP did not get sick or show fever, while unvaccinated controls showed various clinical signs of disease including fever from day 12 post-challenge (dpc) and had to be euthanized at 18 and 20 dpc. These results evidence the protective character of the vaccine formulation of the present invention in the target species. A dose-response relationship is also demonstrated as formulation with higher amounts of E2CD154 and c-di-AMP conferred better immune response and better protection in pigs than formulation with lower doses.

In the examples presented in this invention c-di-AMP was used, but it is evident to a person skilled in the art that other CDN such as for example c-di-GMP or c-di-GAMP, which are also agonists of STING receptors, may have similar effects as immunopotentiators.

In a preferred embodiment of the invention, vaccine compositions comprising the protein identified as SEQ ID NO: 1 and the adjuvant c-di-AMP are administered to pigs using a bait. The efficacy of this form of administration is illustrated in example 5, where a rice-based bait is used, but it is evident to a person skilled in the art that baits constructed from different attractive foods for pigs, such as wheat, corn, or other cereals, can be used with similar results.

Example 6 shows the results of the viral challenge, where it is evidenced that the pigs from the previous example, immunized by means of baits, are protected against CSF. The present invention also shows that the synergistic combination of the molecular antigen CD154 with the adjuvant c-di-AMP can be used for the development of oral vaccines against other pathogens.

In example 7 it is shown how a vaccine formulation formed by one or two proteins of African swine fever virus (ASFV) (p72t and p30) fused to CD154 (SEQ ID NO: 2 and SEQ ID NO: 3), in combination with c-di-AMP, achieves a better humoral and cellular immune response against ASFV when administered orally, compared to p72t protein without CD154 combined with c-di-AMP, or the same proteins administered without c-di-AMP. It is shown that the antibodies generated can neutralize ASFV replication *in vitro,* and that animals vaccinated with these formulations combined with c-di-AMP are protected from disease against a challenge with a virulent strain of ASFV.

### Brief description of the figures

**Figure 1****. Weight of animals immunized orally with E2CD154/c-di-AMP combination and controls receiving only E2CD154 or PBS.** Triangles: group 1, negative control PBS; circles: group 2, E2CD154; squares: group 3, E2CD154 + c-di-AMP. Dav (days after vaccination)
**Figure 2****. Anti E2CD154 titers measured by indirect ELISA in mouse serum after two and three doses.** The titers are expressed as the inverse of the last serum dilution giving a positive signal in ELISA (twice the value of negative control). Group 1, negative control PBS; group 2, E2CD154; group 3, E2CD154 + c-di-AMP.
**Figure 3****. Neutralizing antibody titers anti E2CD154 in mouse serum after two and three doses.** Group 1, negative control PBS; group 2, E2CD154; group 3, E2CD154 + c-di-AMP.
**Figure 4****. Anti E2CD154 antibody response measured by indirect ELISA in immunized mice after three doses.** Group 1: E2 + c-di-AMP; group 2: E2CD154 + c-di-AMP; group 3: E2; group 4: E2CD154; group 5: negative controls. There are significant differences between titers of group 2 and rest (Kruskal Wallis, Dunn, p<0.05).
**Figure 5****. Specific antibodies against E2 in serum of immunized pigs are measured by competition ELISA (PrioCHECK CSFV Ab2.0, Thermo Fischer, USA) after fourth administrations.** Group 1: pigs immunized with lower doses of E2CD154 and c-di-AMP; Group 2: pigs immunized with higher doses of E2CD154 and c-di-AMP; Group 3: negative controls.
**Figure 6****. Anti E2CD154 antibody response measured by indirect ELISA in immunized pigs after four administrations.** Group 1: pigs immunized with lower doses of E2CD154 and c-di-AMP; Group 2: pigs immunized with higher doses of E2CD154 and c-di-AMP; Group 3: negative controls.
**Figure 7****. Weight of vaccinated pigs and controls after challenge.** Group 1: Immunized with lower doses of E2CD154 and c-di-AMP; Group 2: Immunized with higher doses of E2CD154 and c-di-AMP; Group 3: Negative controls.
**Figure 8****. Time course of rectal temperature in pigs challenged with CSFV.** A. Average rectal temperature per group; B. Group 3: controls; C. Group 1: immunized with lower doses of E2CD154 and c-di-AMP; D. Group 2: immunized with higher doses of E2CD154 and c-di-AMP.
**Figure 9****. Time course of the clinical score of pigs challenged with CSFV.** A. Group 1: immunized with lower doses of E2CD154 and c-di-AMP; B. Group 2: immunized with higher doses of E2CD154 and c-di-AMP; C. Group 3: negative controls.
**Figure 10****. Time course of clinical score of pigs challenged with CSFV.** A. Group 1: pigs immunized with baits containing only c-di-AMP; B: Group 2: immunized with baits with E2CD154 and c-di-AMP.
**Figure 11****: Total IgG titers specific against ASFV p72t antigen measured by indirect ELISA.** The titers are expressed as the log2 of the reciprocal of the last dilution at which the OD at 450 nm of the problem sera in the ELISA was twice the OD of the pre-immune sera.
**Figure 12****: Total IgG titers specific against ASFV p30 antigen measured by indirect ELISA.** The titers are expressed as the log2 of the reciprocal of the last dilution at which the OD at 450 nm of the problem sera in the ELISA was twice the OD of the pre-immune sera.

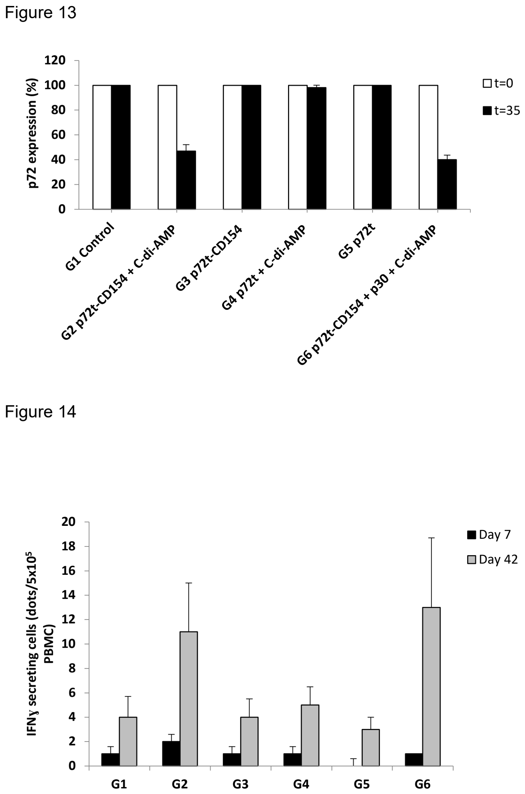

**Figure 13****: Neutralization of ASFV-G infection by serum from animals immunized with different ASFV vaccine candidates.** The ASFV-G strain was incubated with pre-immune sera (white bars) or immune sera (black bars) from the different experimental groups and then Vero cells were infected. After 16 h, the percentage of p72 expression was determined by FACS using a specific MAb against p72. The expression of p72 from Vero cells infected with ASFV-G pre-incubated with pre-immune sera was considered as 100%, against which the % of p72 expression from Vero cells infected with ASFV-G pre-incubated with immune sera was calculated.
**Figure 14****: Induction of IFN-gamma producing cells specific for ASFV antigens.** The number of IFN gamma secreting cells is shown after *in vitro* re-stimulation with the p73t and p30 antigens from PBMC of immunized animals and controls, 7 and 42 days after the first immunization (7 days after the challenge). The data represent the average per group and the standard deviation is represented with bars in the positive direction. The asterisks represent statistically significant differences compared to the control group (ANOVA followed by a Bonferroni multiple comparisons test, (*) p < 0.05; (**) p < 0.001).

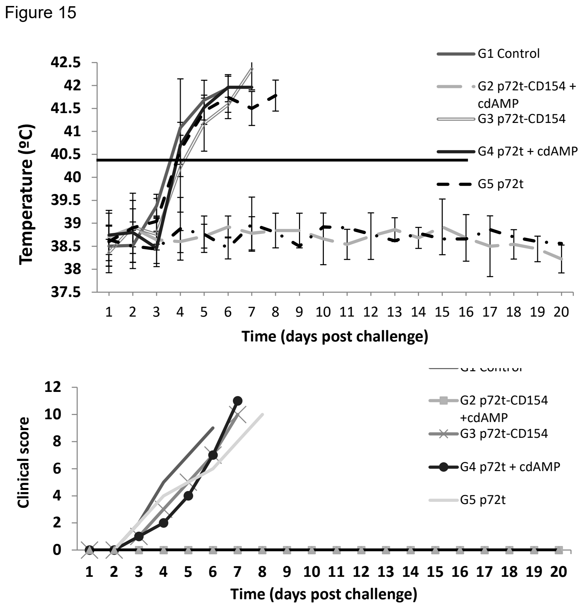

**Figure 15****: Confrontation of immunized pigs and controls with the highly virulent ASFV-G strain.** The challenge took place on day 35 after the first immunization with 10⁴ HAD50 of the ASFV-G strain by intranasal route. (A) Daily rectal temperature of the pigs in each experimental group after viral confrontation. Fever was considered when the rectal temperature was above 40°C for 2 or more consecutive days. The points represent the average temperature per group and the standard deviation is represented by bars in both directions. (B) Clinical score of the pigs in each experimental group after viral confrontation.

### Detailed Exposure of Embodiments / Examples

### Example 1. Immunization of mice by the oral route with the protein identified as SEQ ID NO: 1 in combination with c-di-AMP.

The E2-CD154 protein, identified as SEQ ID NO: 2, was obtained from the HEK 293 cell line that contains the gene encoding this protein (SEQ ID NO: 1) integrated. Cells were cultured in a 10 L BIOSTAT B Plus fermenter (Germany), with serum-free medium. The metabolized culture medium was concentrated in a tangential flow concentrator (PESU 100 kDa cassette, Sartorius, Germany) to an approximate concentration of 0.2 mg/mL and filtered with a 0.2 µM capsule. It was stored at 4°C until use.

A total of 1.6 mg of c-di-AMP (Jenna Bioscience, Germany) were weighed and resuspended in 200 µl of H2O type II for a concentration of 8 mg/mL. Aliquots of 10 µl were made and frozen at -20°C until use.

BALB/c mice between 18 and 22 g in weight (CENPALAB) were immunized. The animals were weighed on day 0 and after each dose. They were divided into 3 groups of 5 mice each. Group 1 received phosphate-buffered saline (PBS) orally; group 2, E2CD154 in phosphate buffer 50 mM + 0.5 M NaCl, pH 7.2, and group 3 was immunized with E2CD154 + c-di-AMP. The mice were immunized on days 0 and 7, blood was drawn on day 15 and a third dose was administered on day 17. The last blood draw was on day 24. In the first administration, 25 µg of E2CD154 and 16 µg of c-di-AMP were given sublingually. In the second and third administrations, 2.5 µg of E2CD154 and 5 µg of c-di-AMP in a volume of 20 µL were given by the same route.

To evaluate the presence of anti-E2CD154 antibodies in the serum of inoculated mice, an indirect ELISA was performed. Maxisorp plates (Thermo Fisher Scientific, USA) were coated overnight with 5 µg/mL of E2CD154 (Lot 2117). It were blocked for 1 h with PBS + 1% skim milk for 1 h at 37°C, and serum samples diluted from 1:40 to1:80 in PBS milk at 1% + 0.05% tween20 were added. A serum from mouse previously immunized with Porvac^{®} vaccine by the intramuscular route was used as positive control in the assay. Sera from non-immunized mice were used as negative control. The sera were incubated for 1 h at 37°C. Plates were washed then twice with PBS + tween 20 0.05% and a 1:5000 dilution of an anti-mouse IgG peroxidase conjugate (Sigma, USA) was added. After 1 h of incubation at 37°C, plates were washed and the chromogenic substrate tetramethylbenzidine (TMB), together with H₂O₂ were added. The reaction was stopped after 10 min with the stop solution (H₂SO₄ 0.2 M) and the optical density at 450 nM was measured in an ELISA plate. The sera with optical density values two times or higher than the negative control were considered positive and titrated through double serial dilutions. The titer was defined as the last dilution of the serum with an OD twice that of the negative control serum at the same dilution.

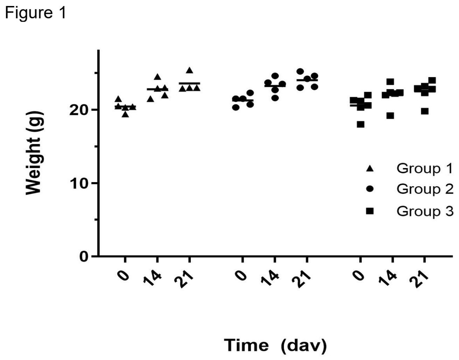

Figure 1 shows that mice immunized with the combination of E2CD154 and c-di-AMP experienced a weight gain similar to control mice. No statistically significant differences with respect to the groups receiving PBS or E2CD154 were found (Student's t-test, p>0.05). The immunogen was well tolerated. No adverse effects were observed in the vaccinated animals.

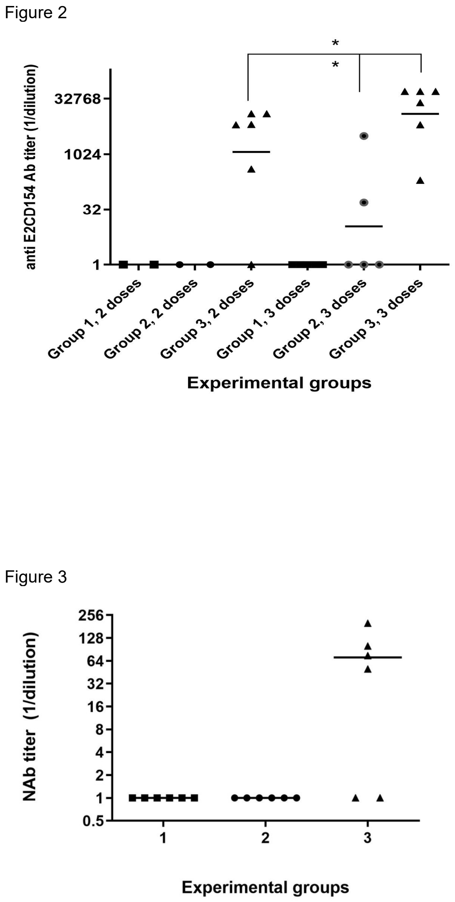

Figure 2 shows the antibody titers against E2CD154 from individual mice measured by ELISA after two and three doses.

After the second administration, five out of six mice immunized with the combination E2CD154 and c-di-AMP had seroconverted, while in the control group, and the two animals evaluated in the E2CD154 group did not show antibodies against E2CD154.

After the third dose, the six mice immunized with the E2CD154 and c-di-AMP mixture had developed antibodies. The geometric mean of the titers increased to 12800, significantly higher than those measured after two doses (Student's T-test, p = 0.0197). Only two out of five mice in the group immunized with E2CD154 alone seroconverted, and with titers significantly lower than those of the combined E2CD514-c-di-AMP group (Student's T-test, p=0.0068). Data in these groups passed the Kolmogorov-Smirnov normality test.

The capacity of the generated antibodies to neutralize CSFV replication was evaluated by NPLA. This technique was developed according to OIE recommendations and as described in previous publications (Santana et al., Int J of Scientific Res in Biol Sciences, 9, 2: 30-34, 2022). Four out of the six mice that responded to the combination of E2CD154/c-di-AMP showed NAb titers in serum above 1:50 (Figure 3). Titers above this value are considered protective in pigs. The mice in the rest of the groups did not show neutralizing titers.

### Example 2. Comparison between the immunogenicity of E2 and E2CD154 together with c-di-AMP by the oral route in mice

To evaluate the individual contribution of c-di-AMP adjuvant and CD154 to the immunogenicity of the oral formulation, BALB/c mice between 18 and 20 g in weight were immunized with E2 or E2CD154 proteins, with and without c-di-AMP. The following experimental groups were formed, each with 5 animals: group 1: 2.5 µg of E2 + 5 µg of c-di-AMP; group 2: 2.5 µg of E2CD154 + 5 µg of c-di-AMP; group 3: 2.5 µg of E2, group 4: 2.5 µg of E2CD154; group 5: 5 µg of c-di-AMP (negative control). The mixture of antigens and adjuvant was formulated in sodium phosphate buffer pH 7.2 + 0.5 M NaCl. The immunogens were administered orally in a volume of 20 µl on days 0, 7 and 14. The presence of specific antibodies against E2 in mouse serum was evaluated 7 days after the last immunization by an indirect ELISA, as described in example 1.

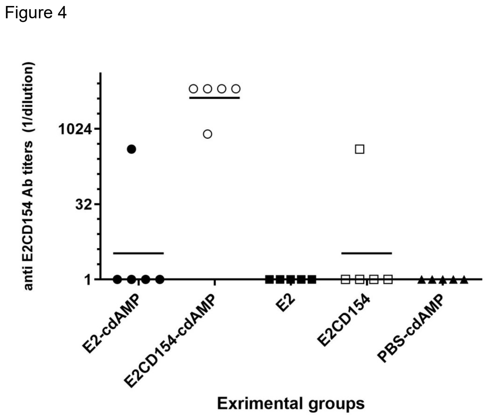

As seen in Figure 4, the best antibody response against E2CD154, both in the frequency of response (100%), and in titers obtained, was found in the group that received the combination E2CD154 with c-di-AMP. Only one mouse out of the five evaluated seroconverted in groups immunized with E2 + c-di-AMP or E2CD154 (groups 1 and 4 respectively). In these two groups NAb titers were much lower than those of group 2. Differences between titers from group 2 and the rest of the groups were statistically significant according to Kruskal Wallis test and Dunn's multiple comparison test for p<0.05. On the other hand, none of the 5 mice evaluated from the group that received protein E2 without adjuvant showed detectable antibodies against E2 (group 3). In addition, mice immunized only with c-di-AMP were also negative (group 5).

These results evidence the synergistic effect of combining molecular adjuvant CD154 with c-di-AMP, which enhances the immunogenicity of protein E2 administered orally. An immune response cannot be observed when either component is administered separately. In no case, a significant response level is achieved.

### Example 3. Immunization of pigs by the oral route with the protein identified as SEQ ID NO: 1 in combination with c-di-AMP.

The E2CD154 protein and c-di-AMP were used as described in example 1. Hybrid Duroc/Yorkshire pigs between 9 and 11 Kg in weight (CENPALAB) were used.

The pigs received four immunizations on days 0, 7, 21 and 28 by atomization in the oral cavity as follows: group 1, administrations 1 and 2: 100 µg of E2CD154 + 50 µg of c-di-AMP in 0.75 ml of 50 mM phosphate buffer + 0.5 M NaCl, pH 7.2; administrations 3 and 4: 100 µg of E2CD154 + 200 µg of c-di-AMP in 0.75 ml of phosphate buffer 50 mM + 0.5 M NaCl, pH 7.2; group 2, administrations 1 and 2: 500 µg of E2CD154 + 50 µg of c-di-AMP in 3.6 ml of phosphate buffer 50 mM + 0.5 M NaCl, pH 7.2, administrations 3 and 4: 500 µg of E2CD154 + 400 µg of c-di-AMP in 3.6 ml of phosphate buffer 50 mM + 0.5 M NaCl, pH 7.2; group 3: control with PBS. Blood was drawn on days 15, 28 and 34 from the ophthalmic orbital sinus. Serum was separated and stored at -20°C until use.

The presence of anti-E2CD154 antibodies in pig serum was evaluated in two different ELISA formats: 1) PrioCHECK CSFV Ab2.0 competition ELISA (Thermo Fischer, USA) according to the manufacturer's recommendations and 2) "in house" indirect ELISA. The positivity criterion for sera was an inhibition index equal to or higher than 40%, and an optical density value twice higher than the negative control.

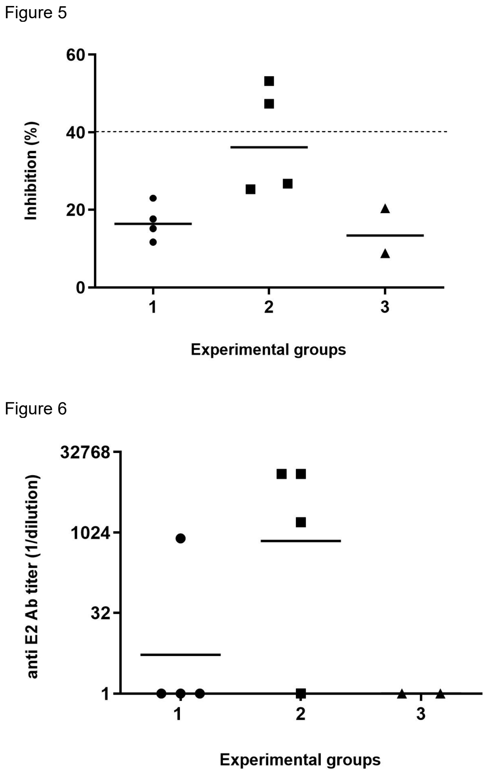

Figure 5 shows the antibody titers against E2 from individual pigs after four inoculations measured in PrioCHECK CSFV Ab2.0 competition ELISA. After the fourth administration, two out of four pigs from group two that received the highest dose of the combination of E2CD154 and c-di-AMP had seroconverted, while the control group and those that received lower doses did not present antibodies against E2CD154.

The indirect ELISA was performed on Polysorp plates (Thermo Fisher, USA) coated with E2CD154 (Batch2117) at a concentration of 5 µg/mL. The plates were blocked for 1 hour with PBS + 1 % skim milk at a temperature of 37°C and serial double dilutions of sera starting from 1:200 were added in PBS + 1 % skim milk + 0.05 % tween 20. As a positive control, serum from a pig immunized intramuscularly with Porvac^{®} vaccine, with a neutralizing titer of 1:50 was used, and as negative controls serum from an unvaccinated pig was used. Sera were incubated for one hour at 37°C. Plates were washed twice with PBS + 0.05 % tween 20, and a 1:10000 dilution of a peroxidase-conjugated anti-pig IgG (Sigma, USA) was added. After 1 h incubation at 37°C, plates were washed and substrate (TMB + H₂O₂) was added. The reaction was stopped with stop solution after 5 minutes and read on an ELISA plate reader at 450 nM. Optical density (OD) values for sera were subtracted from assay blank (wells without serum). Dilutions with an OD value two or more times higher than the negative control and above 0.1 were considered positive. The titer was defined as the last dilution of serum with an OD value higher than 0.1 and more than twice higher than the negative control serum at the same dilution.

Figure 6 shows the antibody titers against E2CD154 in individual pigs after four administrations measured by indirect ELISA. After the fourth administration, three out of four pigs immunized with the mixture of E2CD154 and c-di-AMP had seroconverted. The two sera positive in competition ELISA showed the highest titers. In addition, one out of four pigs from group 1 also seroconverted with a titer of 1:800. The two pigs from the negative control group did not present antibodies against E2CD154.

The presence of CSFV neutralizing antibodies was evaluated by the NPLA assay, performed according to the mentioned procedure (Santana et al., Int. J Scientific Res. Biol. Sciences 2022; 9, 2: 3034). The two sera positive in competition ELISA and with higher titers in indirect ELISA, showed a neutralizing titer of 1:12.5 and 1:25 in this assay.

### Example 4. Protection of vaccinated pigs against challenge with a pathogenic strain of CSFV

Pigs immunized in the previous example were challenged at 34 days post-vaccination (7 days after the last dose) intranasally with 2 x 10³ LD₅₀ of the "Margarita" strain of CSFV. One animal from group 1 was not challenged because it had an accident during transportation to the challenge area, and it was excluded from the study.

The clinical status of the animals was monitored daily after the challenge. The clinical score for signs of classical swine fever was calculated according to the formula proposed by Mittelholzer (Mittelholzer et al. Vet Microbiol, 2000; 74: 293-308).

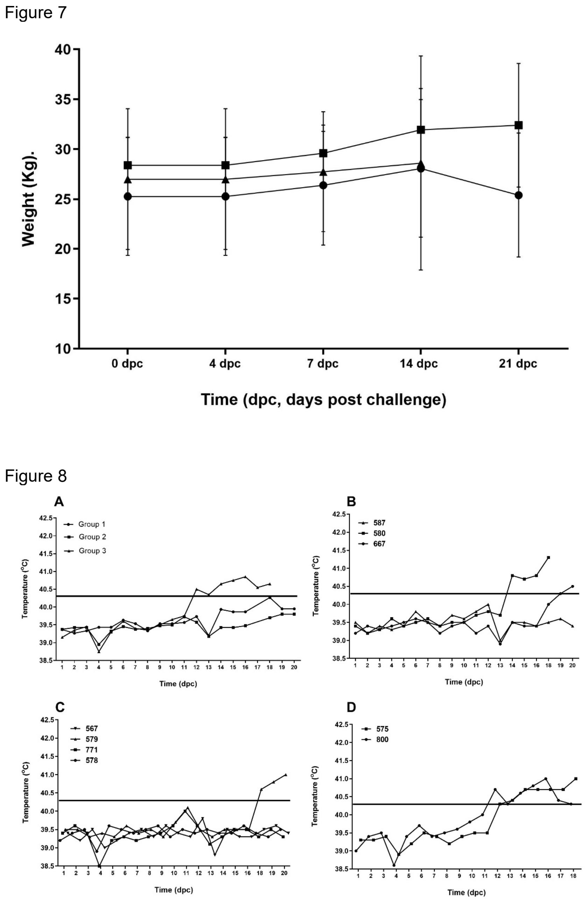

Animals vaccinated with the higher dose (group 2) maintained a tendency to gain weight during the 21 days after challenge, while those vaccinated with the lower dose (group 1) began to lose weight from day 14, and controls (group 3) experienced very low weight gain until their sacrifice at 14 days post-challenge (dpc) (Figure 7).

Figure 8 shows the time course of rectal temperature in the days following challenge. A rectal temperature above 40.3 °C for two consecutive days was considered fever. In Figure 8A it can be seen that the average temperature increased from 12 dpc in controls, and in Figure 8B it is specified that the two pigs in this group debuted with fever at 12 dpc and remained in that state until their sacrifice at 18 and 20 dpc (Figure 8B).

In the group immunized with lower doses of E2CD154 and c-di-AMP, an increase in temperature was observed in one pig from day 14 and another from day 18 that became sustained fever (Figure 8C). The other pig in the group did not experience an increase in rectal temperature. However, in group 1, three pigs vaccinated with the higher doses of ECD154 and c-di-AMP, only one animal showed an increase in temperature with sustained fever from 18 dpc.

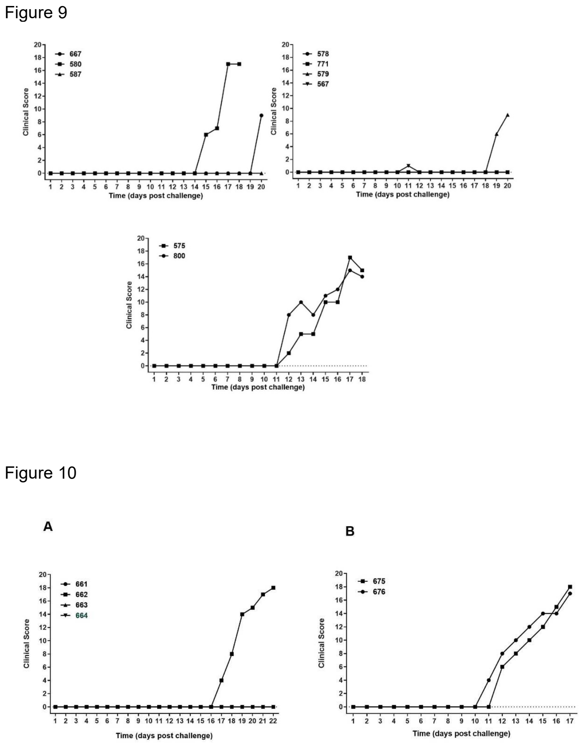

Figure 9 shows the clinical score of animals after challenge. Control animals began to develop clinical signs of disease from 12 dpc and were sacrificed on day eighteen (Figure 9C). Two out of three pigs vaccinated with the lowest dose showed signs of CSF, one from day fifteen and another on day twenty. These animals were sacrificed at 18 and 20 dpc due to advanced CSF clinical signs (Figure 9 A). The third animal in this group remained free from CSF signs. Meanwhile, only one out of four animals immunized with the higher dose showed clinical signs of CSF from 19 dpc and was sacrificed on 20 dpc (Figure 9B). This animal 579 was the only one that had not developed an antibody response against E2 on challenge day, which indicates a correlation between the presence of specific antibodies in serum (even with modest titers) and protection to challenge.

### Example 5. Oral immunization of pigs with E2CD154 administered orally in baits.

To evaluate a formulation and method of administration of the immunogen closer to what is expected to be used in a vaccine intended for wild pigs, baits were prepared with cooked rice. The E2CD154 protein was lyophilized, mixed with c-di-AMP, and included in biodegradable capsules in a proportion of 500 µg of E2CD154 and 500 µg of c-di-AMP per capsule. Each capsule was coated with a paste of cooked rice to form spherical baits with an approximate diameter of 4 cm. The ability of these baits to induce a specific NAb response against CSFV was evaluated in Duroc/Yorkshire hybrid pigs between 9 and 11 kg in weight (CENPALAB). The animals were individualized in quarters, and the baits were administered on an empty stomach for 3 consecutive days at a rate of one bait per animal. The consumption of the baits by the animals was monitored by permanent visual observation during the first 4 h. This procedure was repeated for 3 consecutive weeks, and at the fourth week, blood was drawn from the animals to evaluate the presence of NAb in serum by the NPLA technique, as described in example 1 of the present invention. Four pigs were immunized with the vaccine formulation and another two animals received baits that only contained the c-di-AMP adjuvant.

Three out of four pigs that received the vaccine formulation developed NAb against CSFV, with titers of 1:25 (pig 670) and 1:50 (pigs 671 and 673). In the fourth animal, NAb titers were below 1:12.5 (pig 672). In contrast, none of the animals in the control group developed NAb against CSFV. These results demonstrate that controlled administration of the E2CD154 + c-di-AMP combination in a bait is capable of inducing NAb against CSFV.

### Example 6. Protection of pigs vaccinated with the E2CD154-c-di-AMP combination formulated in rice baits against challenge with CSFV

The pigs immunized in the previous example were challenged intranasally with 2 x 10³ LD₅₀ of the Margarita strain of CSFV. The clinical status of the animals was monitored daily after challenge. The clinical score for signs of swine fever was calculated according to the formula proposed by Mittelholzer (Mittelholzer et al., Vet Microbiol. 2000; 74:293-308).

Figure 10 shows the clinical score of animals after challenge. Control animals began to develop clinical signs of disease from 11 dpc and were sacrificed on 17 dpc (Figure 10A). In contrast, only one out of four vaccinated pigs showed signs of CSF, from day sixteen and was sacrificed at 22 dpc due to advanced CSF clinical signs (Figure 10B). This animal 672 was the only one that had not developed NAb response against E2CD154 on challenge day, which confirms the correlation between the presence of specific antibodies in serum and protection to challenge. The remaining three pigs that exhibited NAb against CSF on challenge day did not show any clinical sign of disease until the end of trial.

The results demonstrate that it is possible to achieve protection in pigs with the combination E2CD154 + c-di-AMP when it is administered as baits orally.

### Example 7. Oral immunization of pigs with a formulation of the antigens of the African swine fever virus identified with SEQ ID NO:2 and SEQ ID NO:3 in combination with c-di-AMP.

With the aim of evaluating the humoral and cellular response generated by vaccine candidates against the African Swine Fever virus (ASFV), crossbred Duroc/Yorkshire pigs of nine weeks of age between 25-30 kg negative for CSF and ASF were immunized with different formulations. The animals were fed 2 kg/animal/day of commercial feed (CENPALAB, Cuba) and water *ad libitum.* All experimental procedures were performed according to guidelines and procedures for the use of laboratory animals. The selected antigens were the p72t and p30 proteins of the ASFV, which were evaluated alone and fused to pig CD154 (p72t-CD154 and p30-CD154). The experimental groups were as follows:
**Group 1** (5 animals): non-immunized controls
**Group 2** (5 animals): orally immunized with four doses of 500 µg of the protein p72t-CD154 + 400 µg c-di-AMP on days 0, 7, 21 and 28 in a total volume of 1 mL of PBS.
**Group 3** (5 animals): orally immunized with four doses of 500 µg of the protein p72t-CD154 on days 0, 7, 21 and 28 in a total volume of 1 mL of PBS.
**Group 4** (5 animals): orally immunized with four doses of 500 µg of the protein p72t + 400 µg c-di-AMP on days 0, 7, 21 and 28 in a total volume of 1 mL of PBS.
**Group 5** (5 animals): orally immunized with four doses of 500 µg of the protein p72t on days 0, 7, 21 and 28 in a total volume of 1 mL of PBS.
**Group 6** (5 animals): orally immunized with four doses of 500 µg of proteins p72t-CD154 and p30-CD154 + 400 µg c-di-AMP in a total volume of 1 mL PBS on days 0, 7, 21 and 28.

Blood was drawn from the animals every 7 days to determine antibody titers. The titer of total IgG specific against viral antigens p72t and p30 was determined by an indirect ELISA developed in our laboratory. For this purpose, "High binding" plates (Costar, USA) were coated with 100 µL of p72t or p30 at a concentration of 10 µg/mL overnight at 4°C. Then, 4 washes were performed with PBS + 0.05% Tween 20. These plates were blocked with 3% skim milk in PBS for 1 h at 37°C, and then 100 µL of serial double dilutions in PBS from the sera under study were applied. Sera were previously inactivated at 56°C for 30 min, starting with a 1:500 dilution and up to 1:64000. Plates were incubated at 37°C for 1 h, and then 4 washes were performed with PBS + 0.05% Tween 20. Subsequently, 100 µL of a 1:10000 dilution of anti-pig IgG conjugated to peroxidase (Sigma, USA) was added. Plates were incubated again at 37°C for 1 h. After 4 washes under the same conditions as before, the reactions were revealed using a substrate solution with 0.1 % tetramethylbenzidine (TMB, 1 mg/mL in DMSO) in substrate buffer (0.5 M citric acid + 0.2 M Na₂HPO₄, pH 5.0), and 0.05 % hydrogen peroxide for 10 min at room temperature. After stopping the reaction with 50 µL of 2 M H₂SO₄, absorbances from the wells were measured at 450 nm in a plate reader (SUMA, Immunoassay Center, Cuba). The titer was defined as the highest serum dilution where absorbance was greater than or equal to twice the absorbance of pre-immune serum.

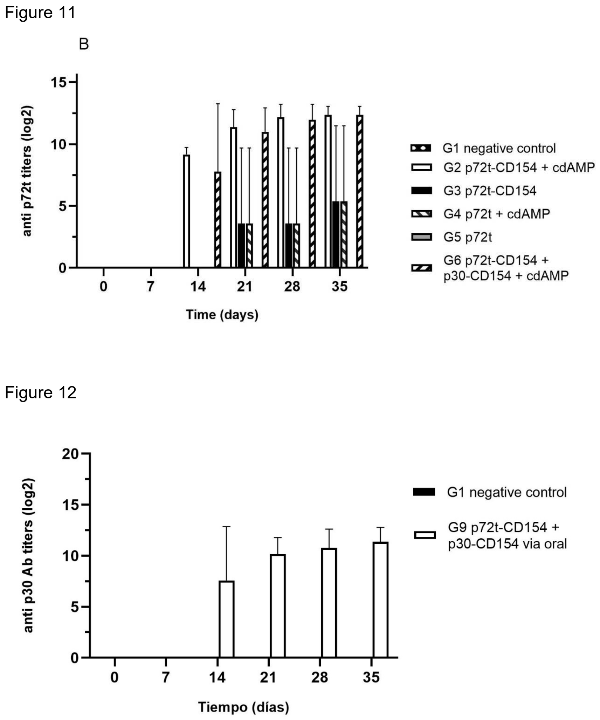

Antibody titers against p72t were observed only when the antigen was the chimeric protein p72t-CD154 combined with c-di-AMP, regardless whether it was combined or not with p30-CD154 (groups 2 and 6). There were no differences in the anti-p72t IgG titers between these two groups (Figure 11).

Animals from group 6 also developed specific IgG titers against p30 starting from day 14 (Figure 12).

The neutralizing capacity of these antibodies against the ASFV was tested by FACS (Fluorescence Activating Cell Sorting). For this, monolayers of Vero cells cultured in DMEM (Dulbecco's modified Eagle's medium) with 5% (v/v) fetal bovine serum and gentamicin at 100 µg/mL were infected with an MOI of 1 using a viral strain multiplied in these cells with less than 15 passages, derived from the highly virulent parental virus of the Georgia/2007 isolate (ASFV-G). This viral strain was previously incubated for 18 h at 37°C with a 1:5 dilution of pre-immune or immune sera on the day of challenge, respectively. The percentage of cells expressing the main viral capsid protein p72t was determined 16 h after infection using a monoclonal antibody against this protein conjugated to FITC at a dilution of 1:10 by flow cytometry on a CyFlow Space Cytometer (Partec) equipped with the FloMax^{®} program. Each sample was tested in duplicate. Vero cells without the addition of the conjugated MAb were used as unmarked controls to determine the base signal of autofluorescence from the samples. The analysis of the acquired data was performed with the FlowJo v.10 program (FlowJo LLC, Tree Star). The statistical analysis of the percentages was performed using the GraphPad Prism version 8.0 program (GraphPad Software).

As seen in Figure 13, the highest percentages of inhibition of p72t expression by Vero cells infected with ASFV were obtained when CD154 and c-di-AMP were included along with antigens in vaccination.

For the study of cellular response, 7 days after the first immunization and 7 days after challenge, peripheral blood mononuclear cells (PBMC) were isolated from 3 animals from each group by centrifugation with Ficoll (GE Healthcare Life Sciences), and resuspended in RPMI 1640 medium supplemented with 1X antibiotic antimycotic solution and 10% fetal bovine serum. The antigen-specific IFN-γ response was determined by an ELISpot assay (Enzyme-Linked ImmunoSpot Assay) BASIC IFN-gamma porcine (MABTECH).

The assay was performed using 96-well plates according to the manufacturer's instructions. Briefly, plates were coated overnight at 4°C with 100 µL of anti-IFN-γ capture antibody prepared at 10 µg/mL. Then, plates were washed five times with 200 µL of sterile PBS and blocked with culture medium for 2 h at 37°C. Then, each well was added with 5 x 10⁵ PBMC from each sample in triplicate, previously mixed with 5 µg/mL of the viral proteins p72t and p30. As controls, cells were added in absence of virus (negative control), or were mixed with the mitogen concanavalin A (Con A) at 6 µg/mL as positive control. After 36 h incubation at 37°C, plates were washed 5 times with 200 µL per well of PBS, and then, detection antibody anti-IFN-γ biotinylated (P2C11) at 0.5 µg/mL in PBS + 0.5% fetal calf serum (FCS) was added for 2 h at room temperature. Subsequently, plates were washed and streptavidin-alkaline phosphatase diluted 1:1000 in PBS with 0.5% FCS was added. After 1 hour incubation at room temperature, plates were washed again with PBS and revealed with 100 µL per well of the BCIP/NBT substrate (Mabtech, # cat 365 zero - ten) for 30 min. The reaction was stopped by washing the plate with tap water. Plates were left to dry overnight and spots were counted. To calculate the number of antigen-specific IFN gamma producing cells in each sample, the number of spots obtained in PBMC stimulated with viral proteins was subtracted from the number of spots obtained in absence of these.

The results showed that seven days after the challenge, a significant stimulation of IFN gamma production was evidenced only in those groups where the ASFV antigens were combined with CD154 and c-di-AMP. In the case of the group immunized with the chimeric p72t-CD154 protein combined with p30-CD154 and c-di-AMP, the stimulation of IFN gamma production was significantly higher than that of the group immunized with the chimeric p72t-CD154 protein and c-di-AMP (Figure 14).

The challenge of all experimental groups was performed under biosafety level 3 containment conditions. Pigs were inoculated 7 days after the last immunization, by instilling in each animal 1 mL containing 10⁴ HAD₅₀ (median hemadsorption units) of the ASFV-G strain intranasally. The temperature (rectal) was recorded daily for a period of 20 days after challenge, as well as clinical signs such as anorexia, depression, purple discoloration of the skin, staggering gait, diarrhea, and cough. Samples (blood and oral swabs) were collected on days 2, 4, 8, 16 and 28 post-inoculation. These samples were used to detect the presence of the virus by qPCR technique. In parallel, virus replication in Vero cell cultures was detected by flow cytometry, as previously described.

From the fourth day post-challenge, control group animals developed clinical signs compatible with ASF such as fever with temperature values above 41°C (Figure 14A), anorexia and ataxia. From the sixth day of viral confrontation, a clear deterioration of animal health was observed that included diarrhea, conjunctivitis, respiratory disorders, severe prostration and nervous symptoms, so animals in this group were sacrificed for ethical reasons. Necropsies revealed specific lesions of ASF infection. The groups immunized with preparations that did not contain CD154 and c-di-AMP along with antigens in the vaccine formulation showed the same clinical signs as control animals and were sacrificed between 6 dpc and 8 dpc. Animals immunized with chimeric p72t-CD154, combined or not with p30-CD154 + c-di-AMP were able to overcome the challenge without clinical manifestations of disease (Figure 14B). No macroscopic pathological lesions compatible with ASF were observed at 28 dpc when all animals were sacrificed.

The presence of virus in blood and oral swabs after challenge was demonstrated in control group animals and those receiving antigens that did not simultaneously contain CD154 and c-di-AMP. Blood samples and oral swabs from groups 2 and 6 were negative for ASF both by PCR and FACS at all times studied (Table 1).

**Table 1. qPCR results from blood samples and oral swaps from pigs in each experimental group after the viral challenge.**

| | dpc | 2 | | 4 | | 8 | | 16 | | 28 | |
|---|---|---|---|---|---|---|---|---|---|---|---|
| | Samples | Blood | Swaps | Blood | Swaps | Blood | Swaps | Blood | Swaps | Blood | Swaps |
| G1 | 1.1 | Neg | Pos | pos | Pos | | | | | | |
| Control | 1.2 | Pos | Pos | Pos | Pos | | | | | | |
| | 1.3 | Neg | Pos | Pos | Pos | | | | | | |
| | 1.4 | Neg | Pos | Pos | Pos | | | | | | |
| | 1.5 | Pos | Pos | Pos | Pos | | | | | | |
| G2 | 5.1 | Neg | Neg | Neg | Neg | Neg | Neg | Neg | Neg | Neg | Neg |
| p72t-CD154 + c-di-AMP | 5.2 | Neg | Neg | Neg | Neg | Neg | Neg | Neg | Neg | Neg | Neg |
| | 5.3 | Neg | Neg | Neg | Neg | Neg | Neg | Neg | Neg | Neg | Neg |
| | 5.4 | Neg | Neg | Neg | Neg | Neg | Neg | Neg | Neg | Neg | Neg |
| | 5.5 | Neg | Neq | Neg | Neg | Neg | Neg | Neg | Neg | Neg | Neg |
| G3 | 6.1 | Neg | Pos | Pos | Pos | | | | | | |
| p72t-CD154 | 6.2 | Neg | Pos | Pos | Pos | | | | | | |
| | 6.3 | Neg | Pos | Neg | Pos | | | | | | |
| | 6.4 | Neg | Pos | Pos | Pos | | | | | | |
| | 6.5 | Neg | Pos | Pos | Pos | | | | | | |
| G4 | 7.1 | Neg | Pos | Pos | Pos | | | | | | |
| p72t + c-di-AMP | 7.2 | Pos | Pos | Pos | Pos | | | | | | |
| | 7.3 | Pos | Pos | Pos | Pos | | | | | | |
| | 7.4 | Neg | Pos | Pos | Pos | | | | | | |
| | 7.5 | Neg | Pos | Pos | Pos | | | | | | |
| G5 | 8.1 | Pos | Pos | Pos | Pos | Pos | Pos | | | | |
| p72t | 8.2 | Pos | Pos | Pos | Pos | Pos | Pos | | | | |
| | 8.3 | Pos | Pos | Pos | Pos | Pos | Pos | | | | |
| | 8.4 | Pos | Pos | Pos | Pos | Pos | Pos | | | | |
| | 8.5 | Neg | Pos | Neg | Pos | Pos | Pos | | | | |
| G6 | 9.1 | Neg | Neg | Neg | Neg | Neg | Neg | Neg | Neg | Neg | Neg |
| p72t-CD154 + p30 +c-di-AMP | 9.2 | Neg | Neg | Neg | Neg | Neg | Neg | Neg | Neg | Neg | Neg |
| | 9.3 | Neg | Neg | Neg | Neg | Neg | Neg | Neg | Neg | Neg | Neg |
| | 9.4 | Neg | Neg | Neg | Neg | Neg | Neg | Neg | Neg | Neg | Neg |
| | 9.5 | Neg | Neg | Neg | Neg | Neg | Neg | Neg | Neg | Neg | Neg |

The blank cells in the table correspond to pigs that showed a marked health deterioration and were sacrificed by ethical reasons.

## Claims

1. A vaccine formulation **characterized in that** it is an oral vaccine comprising: i) an immunologically relevant, chimeric antigen from a swine pathogenic microorganism fused to the CD154 molecule of pigs, ii) an adjuvant selected from the family of cyclic di-nucleotide monophosphates, and iii) an excipient acceptable for oral immunization.

2. The vaccine formulation of claim 1 where the antigen is the chimeric protein comprising the extracellular segment of the CSFV E2 protein and the pig CD154 molecular adjuvant, whose amino acid sequence consists of SEQ ID NO: 1.

3. The vaccine formulation of claim 1 where the antigen includes a combination of two chimeric proteins comprising the p72t and p30 proteins of ASFV, both fused to the pig CD154 molecular adjuvant, and whose amino acid sequences consist of SEQ ID NO: 2 and SEQ ID NO: 3.

4. The vaccine formulation of claim 1 where the adjuvant selected from the family of cyclic di-nucleotide monophosphates is cyclic di-adenosine monophosphate (c-di-AMP).

5. The vaccine formulation of claim 4 that is formulated for use with some cereal as bait.

6. The vaccine formulation of claim 5 in which the bait is made from rice.

7. The vaccine formulation of claim 1 containing between 100 µg and 1000 µg of the chimeric protein, and between 100 µg and 2000 µg of the adjuvant from the family of cyclic di-nucleotide monophosphates.

8. A method for inducing an immune response against classical swine fever virus (CSFV) **characterized in that** a vaccine formulation comprising a chimeric protein formed by i) the extracellular segment of CSFV E2 protein and pig CD154 molecular adjuvant, and ii) an adjuvant selected from the family of cyclic di-nucleotide monophosphates is administered to a pig.

9. The method of claim 8 where the vaccine formulation is administered as bait.

10. A method for inducing an immune response against African swine fever virus (ASFV) **characterized in that** a vaccine formulation comprising: i) chimeric proteins p72t and p30 from ASFV fused to pig CD154 molecular adjuvant, and ii) an adjuvant selected from the family of cyclic di-nucleotide monophosphates is administered orally to a pig.

11. The method of claim 10 where the vaccine formulation is administered as bait.
